# EUROPEAN PATENT SPECIFICATION

(11) **EP 2 295 545 B1**
(45) Date of publication and mention of the grant of the patent: **29.04.2015**
(21) Application number: 10180881.4
(22) Date of filing: 11.06.2003
(51) Int. Cl.: C12N 9/02, C12P 7/06, C12G 3/02, C12N 1/38, C12P 7/10

(54) **Fermentation methods and compositions**
Fermentationsverfahren und Zusammensetzungen
Procédés et compositions de fermentation

(30) Priority: 26.09.2002 US 413730 P
(43) Date of publication of application: 16.03.2011
(62) Divisional of application: 07121010.8
(73) Proprietor: Novozymes North America, Inc., Franklinton, North Carolina 27525 (US)
(72) Inventor: Grichcko, Varvara, Raleigh, NC 27614 (US)
(74) Representative: Shenker, Paul Dhan

(56) References cited:
- EP-A1- 1 122 303
- JP-A- 6 090 734
- JP-A- 10 028 516
- LARSSONS ET AL: "Comparison of Different Methods for the Detoxification of Lignocellulose Hydrolyzates of Spruce", APPLIED BIOCHEMISTRY AND BIOTECHNOLOGY, THE HUMANA PRESS, INC, US, vol. 77-79, 1 January 1999 (1999-01-01), pages 91-103, XP007904792, ISSN: 0273-2289, DOI: DOI:10.1385/ABAB:77:1-3:91
- KLOSEIKO: "Relationship of whole tree carbohydrates and treebiomass in Norway spruce with the wood ash treated soil", FORESTRY STUDIES, vol. 41, 2004, pages 42-52,

## Description

### FIELD OF THE INVENTION

The present invention relates to enzymatic methods and compositions for producing fermentation products, including, methods and compositions for improving yeast performance during fermentation processes.

### BACKGROUND OF THE INVENTION

Fermentation processes are used for making a vast number of commercial products, including alcohols (e.g., ethanol, methanol, butanol); organic acids (e.g., citric acid, acetic acid, itaconic acid, lactic acid, gluconic acid); ketones (e.g., acetone); amino acids (e.g., glutamic acid); gases (e.g., H₂ and CO₂), and more complex compounds, including, for example, antibiotics (e.g., penicillin and tetracycline); enzymes; vitamins (e.g., riboflavin, B₁₂, beta-carotene); hormones, and other compounds which are difficult to produce synthetically. Fermentation processes are also commonly used in the consumable alcohol (e.g., beer and wine), dairy (e.g., in the production of yogurt and cheese), leather, and tobacco industries.

EP1122303A1 disclose a process for producing beer having an improved flavour stability by adding an oxygen scavenging such as laccase enzyme prior to and/or during the mashing stage of the brewing.

There is a need for further improvement of fermentation processes and for improved processes which include a fermentation step.

### SUMMARY OF THE INVENTION

Methods and compositions for producing a fermentation product are disclosed. Methods and compositions for improving yeast performance during fermentation processes, including the rate and/or yield of the fermentation process are disclosed. Methods and compositions for producing ethanol are disclosed.

One aspect of the invention provides a method for producing ethanol from starch-containing material, which method comprises a fermentation step, comprising contacting a fermenting microorganism or fermentation media used in the fermentation step with at least one laccase (EC 1.10.3.2), wherein the starch-containing material is selected from the group consisting of tubers, roots, whole grains, corns, cobs, wheat, barley, rye, milo or cereals. The laccase is applied in an effective amount during fermentation and/or the laccase is applied in an effective amount before fermentation, such as, during the propogation of the fermenting microorganisms. Although not limited to any one theory of operation, it is believed that the use of at least one laccase in the fermentation process promotes the oxidation of inhibitors and oxygen depletion, so as to promote the creation of an anaerobic environment more suitable to the fermenting microorganism.

In a preferred embodiment, the fermentation processes of the present invention are used in combination with a liquefaction process and/or saccharification process, in which additional enzymatic activities, such as, alpha-amylase and glucoamylase activity are used in processing the starch substrate.

Yet another aspect of the present invention relates to the addition of stimulators for growth of the fermenting microorganism in combination with the enzymatic processes described herein, to further improve the fermentation process. Preferred stimulators for growth include vitamins and minerals.

### DETAILED DESCRIPTION OF THE INVENTION

"Fermentation" or "fermentation process" refers to any fermentation process or any process comprising a fermentation step used to produce ethanol. Fermentation processes also include fermentation processes used in the consumable alcohol industry (e.g., beer and wine).

"Fermentation media" or "fermentation medium" refers to the environment in which the fermentation is carried out and which includes the fermentation substrate, that is, the carbohydrate source (i.e. starch-containing material selected from the group consisting of tubers, roots, whole grains, corns, cobs, wheat, barley, rye, milo and cereals that is metabolized by the fermenting microorganism. The fermentation media, including fermentation substrate and other raw materials used in the fermentation process may be processed, e.g., by milling, liquefaction and saccharification processes or other desired processes prior to or simultaneously with the fermentation process. Accordingly, the fermentation media can refer to the media before the fermenting microorganisms are added, such as, the media in or resulting from a liquefaction or saccharification process, as well as the media which comprises the fermenting microorganisms, such as, the media used in a simultaneous saccharification and fermentation process (SSF).

The fermentation media or fermentation substrate may also comprise de-germed cereal grain, preferably de-germed corn, i.e. endosperm and torrified corn (e.g., heat-treated and poped corn) or any other cereal. The cereal grain can be ground or it can be fermented as whole.

"Fermenting microorganism" refers to any microorganism suitable for use in a desired fermentation process for producing ethanol. Suitable fermenting microorganisms according to the invention are able to ferment, i.e., convert, sugars, such as glucose or maltose, directly or indirectly into ethanol. Examples of fermenting microorganisms include fungal organisms, such as yeast. Preferred yeast include strains of the *Sacchromyces* spp., and in particular, *Sacchromyces cerevisiae.* Commercially available yeast include, e.g., Red Star®/Lesaffre Ethanol Red (available from Red Star/Lesaffre, USA) FALI (available from Fleischmann's Yeast, a division of Burns Philp Food Inc., USA), SUPERSTART (available from Alltech), GERT STRAND (available from Gert Strand AB, Sweden) and FERMIOL (available from DSM Specialties).

The substrates for use in the processes of present invention are tubers, roots, whole grains, corns, cobs, wheat, barley, rye, milo and cereals.

The fermentation processes described herein are preferably used in combination with liquefaction or saccharification processes. Any liquefaction or saccharification may be used in combination with the fermentation process of the present invention. According to the present invention, the saccharification and liquefaction may be carried out simultaneously or separately with the fermentation process. In a preferred embodiment of the present invention, the liquefaction, saccharification and fermentation processes are carried out simultaneously.

"Liquefaction" is a process in which milled (whole) grain raw material is broken down (hydrolyzed) into maltodextrins (dextrins). Liquefaction is often carried out as a three-step hot slurry process. The slurry is heated to between 60-95°C, preferably 80-85°C, and the enzymes are added to initiate liquefaction (thinning). The slurry is then jet-cooked at a temperature between 95-140°C, preferably 105-125°C to complete gelatinization of the slurry. Then the slurry is cooled to 60-95°C and more enzyme(s) is(are) added to finalize hydrolysis (secondary liquefaction). The liquefaction process is usually carried out at pH 4.5-6.5, in particular at a pH between 5 and 6. Milled and liquefied whole grains are known as mash.

The liquefaction processes are typically carried out using an alpha-amylase. Preferred alpha-amylases are of fungal or bacterial origin. More preferably, the alpha-amylase is a *Bacillus* alpha-amylases, such as, derived from a strain of *B. licheniformis, B. amyloliquefaciens,* and *B. stearothermophilus.* Other alpha-amylases include alpha-amylase derived from a strain of the *Bacillus* sp. NCIB 12289, NCIB 12512, NCIB 12513 or DSM 9375, all of which are described in detail in WO 95/26397, and the alpha-amylase described by Tsukamoto et al., Biochemical and Biophysical Research Communications, 151 (1988), pp. 25-31. Other alpha-amylase variants and hybrids are described in WO 96/23874, WO 97/41213, and WO 99/19467. Other alpha-amylase include alpha-amylases derived from a strain of *Aspergillus,* such as, *Aspergillus oryzae* and *Aspergillus niger* alpha-amylases.

In a preferred embodiment, the alpha amylase is an acid alpha amylases. The term "acid alpha-amylase" means an alpha-amylase (E.C. 3.2.1.1) which when added in an effective amount has activity at a pH in the range of 3.0 to 7.0, preferably from 3.5 to 6.0, or more preferably from 4.0-5.0. Any suitable acid alpha-amylase may be used in the present invention.

In a preferred embodiment, the acid alpha-amylase is an acid fungal alpha-amylase or an acid bacterial alpha-amylase. Preferred acid alpha-amylase for use in the present invention may be derived from a strain of *B. licheniformis, B. amyloliquefaciens,* and *B. Stearothermophilus.* More preferably, the acid alpha-amylase is the an acid fungal alpha amylases, such as, SP 288 (available from Novozymes).

Preferred commercial compositions comprising alpha-amylase include MYCOLASE (Gist Brocades), BAN™, TERMAMYL™ SC, FUNGAMYL™, LIQUOZYME™ X and SAN™ SUPER, SAN™ EXTRA L (Novozymes A/S) and CLARASE L-40,000, DEX-LO™, SPEYME FRED, SPEZYME™ AA, and SPEZYME™ DELTA AA (Genencor Int.).

The alpha-amylase may be added in amounts as are well-known in the art. When measured in AAU units the acid alpha-amylase activity is preferably present in an amount of 5-500000 AAU/kg of DS, in an amount of 500-50000 AAU/kg of DS, or more preferably in an amount of 100-10000 AAU/kg of DS, such as 500-1000 AAU/kg DS. Fungal acid alpha-amylase are preferably added in an amount of 10-10000 AFAU/kg of DS, in an amount of 500-2500 AFAU/kg of DS, or more preferably in an amount of 100-1000 AFAU/kg of DS, such as approximately 500 AFAU/kg DS.

"Saccharification" is a process in which the maltodextrin (such as, produced from the liquefaction process) is converted to low molecular sugars DP₁₋₃(i.e., carbohydrate source) that can be metabolized by the fermenting organism, such as, yeast. Saccharification processes are well known in the art and are typically performed enzymatically using a glucoamylase. Alternatively or in addition, alpha-glucosidases or acid alpha-amylases may be used. A full saccharification process may last up to from about 24 to about 72 hours, and is often carried out at temperatures from about 30 to 65 degrees Celsius, and at a pH between 4 and 5, normally at about pH 4.5. However, it is often more preferred to do a pre-saccharification step, lasting for about 40 to 90 minutes, at temperature of between 30-65°C, typically about 60°C, followed by complete saccharification during fermentation in a simultaneous saccharification and fermentation process (SSF).

The glucoamylase used in the saccharification process may be derived from any suitable source, e.g., derived from a microorganism or a plant. Preferred glucoamylases are of fungal or bacterial origin, selected from the group consisting of *Aspergillus* glucoamylases, in particular *A. niger* G1 or G2 glucoamylase (Boel et al. (1984), EMBO J. 3 (5), p. 1097-1102), or variants thereof, such as disclosed in WO 92/00381 and,WO 00/04136; the *A. awamori* glucoamylase (WO 84/02921), A. oryzae (Agric. Biol. Chem. (1991), 55 (4), p. 941-949), or variants or fragments thereof.

Other *Aspergillus* glucoamylase variants include variants to enhance the thermal stability, such as, G137A and G139A (Chen et al. (1996), Prot. Engng. 9, 499-505); D257E and D293E/Q (Chen et al. (1995), Prot Engng. 8, 575-582); N182 (Chen et al. (1994), Biochem. J. 301, 275-281); disulphide bonds, A246C (Fierobe et al. (1996), Biochemistry, 35, 8698-8704; and introduction of Pro residues in position A435 and S436 (Li et al. (1997), Protein Engng. 10, 1199-1204. Other glucoamylases include *Talaromyces* glucoamylases, in particular, derived from *Talaromyces emersonii* (WO 99/28448), *Talaromyces leycettanus* (US patent no. Re. 32,153), *Talaromyces duponti, Talaromyces thermophilus* (US patent no. 4,587,215). Bacterial glucoamylases contemplated include glucoamylases from the genus *Clostridium,* in particular *C*. *thermoamylolyticum (*EP 135,138*),* and *C*. *thermohydrosulfuricum* (WO 86/01831).

Commercially available compositions comprising glucoamylase include AMG 200L; AMG 300 L; SAN™ SUPER, SAN EXTRA L, SPIRIZYME PLUS and AMG™ E (from Novozymes A/S); AMIGASE™ and AMIGASE™ PLUS (from DSM); OPTIDEX™ 300, G-ZYME™ G900, G-ZYME™ and G990 ZR (from Genencor Int.).

Glucoamylases may in an embodiment be added in an amount of 0.02-2 AGU/g DS, preferably 0.1-1 AGU/g DS, such as 0.2 AGU/g DS.

The most widely used process in ethanol production is the simultaneous saccharification and fermentation (SSF) process, in which there is no holding stage for the saccharification, meaning that fermenting organism, such as the yeast, and enzyme(s) is(are) added together. In SSF processes, it is common to introduce a pre-saccharification step at a temperature above 50°C, just prior to the fermentation.

More preferably, the liquefaction, saccharification or fermentation process is a simultaneous liquefaction-saccharification-fermentation (LSF) process also referred as a single enzymatic process, in which the liquefaction, saccharification and fermentation process are all carried out in one process, that is, all enzymes (or substitutable or additional non-enzymatic agents) and the yeast used for liquefaction, saccharification and fermentation, accordingly, are added in the same process step, more preferably, simultaneously in the same process step. Preferred process conditions for LSF process include temperatures of about 26°C to 40°C, preferably about 32°C, pH of about 4 to about 8, preferably about pH 5, and process times of about 48 to 72 hours, preferably about 72 hours.

Preferably, the LSF process or single enzymatic process is a raw starch hydrolysis (RSH) process used in the production of ethanol. A "raw starch hydrolysis" process (RSH) differs from conventional starch treatment processes in that raw uncooked starch, also referred to as granular starch, is used in the ethanol fermentation process. As used herein, the term "granular starch" means raw uncooked starch, i.e. starch in its natural form found, e.g., in cereal, tubers or grains. Starch is formed within plant cells as tiny granules insoluble in water. When put in cold water, the starch granules may absorb a small amount of the liquid and swell. At temperatures up to 50°C to 75°C the swelling may be reversible. However, with higher temperatures an irreversible swelling called gelatinization begins.

The term "initial gelatinization temperature" means the lowest temperature at which gelatinization of the starch commences. Starch heated in water begins to gelatinize between 50°C and 75°C; the exact temperature of gelatinization depends on the specific starch, and can readily be determined by the skilled artisan. For example, the initial gelatinization temperature may vary according to the plant species, to the particular variety of the plant species as well as with the growth conditions. In the context of this invention the initial gelatinization temperature of a given starch is the temperature at which birefringence is lost in 5% of the starch granules using the method described by Gorinstein. S. and Lii. C., Starch/Stärke, Vol. 44 (12) pp. 461-466 (1992).

A preferred application of the fermentation processes and compositions described herein is in an ethanol production process, the ethanol being for use as a fuel or fuel additive, more preferably using a raw starch hydrolysis process. The processes described herein can be used, e.g., to increase the rate and/or yield of ethanol production.

Ethanol production processes generally involve the steps of milling, liquefaction, saccharification, fermentation and distillation. In the production of ethanol and other starch-based products, the raw material, such as whole grain, preferably corn, is milled in order to open up the structure and allow for further processing. Two processes are preferred according to the invention: wet milling and dry milling. Preferred for ethanol production is dry milling where the whole kernel is milled and used in the remaining part of the process. Wet milling may also be used and gives a good separation of germ and meal (starch granules and protein) and is with a few exceptions applied at locations where there is a parallel production of syrups. Both wet and dry milling processes are well known in the art.

In ethanol production, the fermenting organism is preferably yeast, which is applied to the mash. A preferred yeast is derived from *Saccharomyces* spp., more preferably, from *Saccharomyces cerevisiae.* In preferred embodiments, yeast is applied to the mash and the fermentation is ongoing for 24-96 hours, such as typically 35-60 hours. In preferred embodiments, the temperature is generally between 26-34°C, in particular about 32°C, and the pH is generally from pH 3-6, preferably around pH 4-5. Yeast cells are preferably applied in amounts of 10⁵ to 10¹², preferably from 10⁷ to 10¹⁰, especially 5x10⁷ viable yeast count per ml of fermentation broth. During the ethanol producing phase the yeast cell count should preferably be in the range from 10⁷ to 10¹⁰, especially around 2 x 10⁸. Further guidance in respect of using yeast for fermentation can be found in, e.g., "The alcohol Textbook" (Editors K. Jacques, T.P. Lyons and D.R.Kelsall, Nottingham University Press, United Kingdom 1999), which is hereby incorporated by reference.

Following fermentation, the mash may be distilled to extract the alcohol product (ethanol). In the case where the end product is ethanol, obtained according to the processes of the invention, it may be used as, e.g., fuel ethanol; drinking ethanol, i.e., potable neutral spirits; or industrial ethanol.

The laccases and other enzymes referenced herein may be derived or obtained from any suitable origin, including, bacterial, fungal, yeast or mammalian origin. The term "derived" or means in this context that the enzyme may have been isolated from an organism where it is present natively, i.e. the identity of the amino acid sequence of the enzyme are identical to a native enzyme. The term "derived" also means that the enzymes may have been produced recombinantly in a host organism, the recombinant produced enzyme having either an identity identical to a native enzyme or having a modified amino acid sequence, e.g. having one or more amino acids which are deleted, inserted and/or substituted, i.e., a recombinantly produced enzyme which is a mutant and/or a fragment of a native amino acid sequence or an enzyme produced by nucleic acid shuffling processes known in the art. Within the meaning of a native enzyme are included natural variants. Furthermore, the term "derived" includes enzymes produced synthetically by, e.g., peptide synthesis. The term "derived" also encompasses enzymes which have been modified e.g. by glycosylation, phosphorylation, or by other chemical modification, whether *in vivo* or *in vitro.* The term "obtained" in this context means that the enzyme has an amino acid sequence identical to a native enzyme. The term encompasses an enzyme that has been isolated from an organism where it is present natively, or one in which it has been expressed recombinantly in the same type of organism or another, or enzymes produced synthetically by, e.g., peptide synthesis. With respect to recombinantly produced enzymes the terms "obtained" and "derived" refers to the identity of the enzyme and not the identity of the host organism in which it is produced recombinantly.

The enzymes may also be purified. The term "purified" as used herein covers enzymes free from other components from the organism from which it is derived. The term "purified" also covers enzymes free from components from the native organism from which it is obtained. The enzymes may be purified, with only minor amounts of other proteins being present. The expression "other proteins" relate in particular to other enzymes. The term "purified" as used herein also refers to removal of other components, particularly other proteins and most particularly other enzymes present in the cell of origin of the enzyme of the invention. The enzyme may be "substantially pure," that is, free from other components from the organism in which it is produced, that is, for example, a host organism for recombinantly produced enzymes. In preferred embodiment, the enzymes are at least 75% (w/w) pure, more preferably at least 80%, at least 85%, at least 90%, at least 95%, at least 96%, at least 97%, at least 98%, or at least 99% pure. In another preferred embodiment, the enzyme is 100% pure.

The enzymes used in the present invention may be in any form (composition) suitable for use in the processes described herein, such as e.g. in the form of a dry powder or granulate, a non-dusting granulate, a liquid, a stabilized liquid, or a protected enzyme. Granulates may be produced, e.g., as disclosed in US Patent Nos. 4,106,991 and US 4,661,452, and may optionally be coated by methods known in the art. Liquid enzyme preparations may, for instance, be stabilized by adding stabilizers such as a sugar, a sugar alcohol or another polyol, lactic acid or another organic acid according to established methods. Protected enzymes may be prepared according to the method disclosed in EP 238,216.

In accordance with another preferred embodiment, a fermentation stimulator may be used in combination with any of the enzymatic processes described herein to further improve the fermentation process, and in particular, the performance of the fermenting microorganism, such as, rate enhancement and ethanol yield. A "fermentation stimulator" refers to stimulators for growth of the fermenting microorganisms, in particular, yeast. Preferred fermentation stimulators for growth include vitamins and minerals. Examples of vitamins include multivitamins, biotin, pantothenate, nicotinic acid, meso-inositol, thiamine, pyridoxine, para-aminobenzoic acid, folic acid, riboflavin, and Vitamins A, B, C, D, and E. *See, e.g*., Alfenore et al., Improving ethanol production and viability of Saccharomyces cerevisia by a vitamin feeding strategy during fed-batch process," Springer-Verlag (2002), which is hereby incorporated by reference. Examples of minerals include minerals and mineral salts that can supply nutrients comprising P, K, Mg, S, Ca, Fe, Zn, Mn, and Cu.

The laccase used in the process of the invention is applied in an effective amount during fermentation and/or before fermentation, such as, during the propogation of the fermenting organisms.

In the context of this invention, laccases comprise any laccase enzyme comprised by the enzyme classification (EC 1.10.3.2).

The above mentioned enzymes may be derived from plants, bacteria or fungi (including filamentous fungi and yeasts) and suitable examples include a laccase derived from a strain of *Aspergillus, Neurospora,* e.g., *N*. *crassa, Podospora, Botrytis, Collybia, Fomes, Lentinus, Pleurotus, Trametes,* e.g., *T. villosa* and *T. versicolor, Rhizoctonia,* e.g., *R*. *solani, Coprinus,* e.g., *C. cinereus, C. comatus, C. friesii,* and *C. plicatilis, Psathyrella,* e.g., *P. condelleana, Panaeolus"* e.g. *P*. *papilionaceus, Myceliophthora,* e.g., *M. thermophila, Schytalidium,* e.g., *S. thermophilum, Polyporus,* e.g., *P. pinsitus, Pycnoporus,* e.g., *P. cinnabarinus, Phlebia,* e.g., *P. radita* (WO 92/01046), or *Coriolus,* e.g., *C. hirsutus* (JP 2-238885).

A laccase derived from *Coprinus, Myceliophthora,* Polyporus, *Pycnoporus, Scytalidium* or *Rhizoctonia* is preferred, in particular a laccase derived from *Coprinus cinereus, Myceliophthora thermophila, Polyporus pinsitus, Pycnoporus cinnabarinus, Scytalidium thermophilum* or *Rhizoctonia solani.*

### EXAMPLE 1

The addition of a laccase was tested under anaerobic fermentation conditions analogous to ethanol fermenting experiments. The experiment was conducted on dry milled yellow corn according to a simultaneous liquefaction-saccharification-fermentation (LSF) protocol. The experiment was carried out using a glucoamylase (Spirizyme plus, available from Novozymes A/S) for fermenting ethanol, with and without laccase. Fermentations were carried out at pH=5.0, 32°C for several hour. The process was monitored with time and analyzed for CO₂ weight loss, percent ethanol produced and theoretical conversion of the starch. As shown in Table 1, the addition of laccase also improved ethanol yield.

**Table 1**

| **Treatment** | **HPLC ethanol, % v/v** | | | | | |
|---|---|---|---|---|---|---|
| | **7h** | **23 h** | **29 h** | **47 h** | **55 h** | **119 h** |
| 0.7 GAU/g DS | 2.36 | 9.29 | 10.55 | 12.38 | 13.13 | 16.73 |
| 0.7 GAU/g DS & 0.1 M NaCl | 1.88 | 7.91 | 8.78 | 10.30 | 10.94 | 14.61 |
| 0.9 GAU/g DS | 2.24 | 9.63 | 10.92 | 12.92 | 13.66 | 18.05 |
| 0.9 GAU/g DS & NZ 525, 0.5% DS | 2.42 | 9.44 | 10.58 | 12.63 | 13.39 | 18.28 |
| 1.0 GAU/g DS | 2.07 | 9.29 | 10.21 | 12.27 | 13.06 | 17.83 |
| 1.0 GAU/g DS & laccase, 0.2% DS | 2.89 | 10.87 | 11.99 | 13.82 | 14.41 | 18.86 |

## Claims

1. A method for producing ethanol from starch-containing material, which method comprises a fermentation step, comprising contacting a fermenting microorganism or fermentation media used in the fermentation step with at least one laccase (EC 1.10.3.2), wherein the starch-containing material is selected from the group consisting of tubers, roots, whole grains, corns, cobs, wheat, barley, rye, milo or cereals.

2. The method of claim 1, wherein said microorganism is a yeast.

3. The method of claims 1 or 2, wherein said fermentation step is part of a simultaneous saccharification and fermentation process.

## Patentansprüche

1. Verfahren zur Herstellung von Ethanol aus stärkehaltigen Materialien, wobei das Verfahren einen Fermentationsschritt einschließt, der das Zusammenbringen eines fermentierenden Mikroorganismus oder eines Fermentationsmediums, das bei dem Fermentationsschritt eingesetzt wird, mit mindestens einer Laccase (EC 1.10.3.2) umfasst, wobei die stärkehaltigen Materialien aus der Gruppe ausgewählt sind, die aus Knollen, Wurzeln, ganze Getreidekörner, Maiskolben, Weizen, Gerste, Roggen, Hirse oder Cerealien besteht.

2. Verfahren nach Anspruch 1, wobei der Mikroorganismus eine Hefe ist.

3. Verfahren nach Anspruch 1 oder 2, wobei der Fermentationsschritt Teil eines gleichzeitigen Saccharifizierungs- und Fermentationsverfahrens ist.

## Revendications

1. Méthode pour produire d'éthanol à partir des matières premières contenant de l'amidon, la méthode comprenant une étape de fermentation comprenant la mise en contact d'un microorganisme fermenteur ou d'un milieu de fermentation utilisé dans l'étape de fermentation avec au moins une laccase (EC 1.10.3.2), dans laquelle les matières premières contenant l'amidon sont sélectionnées parmi le groupe constitué par les tubercules, les racines, les grains entiers, le maïs, les épis de maïs, le blé, l'orge, le seigle, le milo ou les céréales.

2. Méthode selon la revendication 1, dans laquelle ledit microorganisme est une levure.

3. Méthode selon les revendications 1 ou 2, dans laquelle ladite étape de fermentation fait partie d'un procédé de saccharification et de fermentation simultanées.
